# EUROPEAN PATENT APPLICATION

(11) **EP 4 356 832 A1**
(43) Date of publication of application: **24.04.2024**
(21) Application number: 23790532.8
(22) Date of filing: 31.08.2023
(51) Int. Cl.: A61B 5/145, A61B 5/00, A61B 5/02, G06Q 10/04, G06Q 50/22

(54) **SYSTEM AND METHOD FOR PREDICTING BLOOD SUGAR BY USING SALIVA-BASED ARTIFICIAL INTELLIGENCE DEEP-LEARNING TECHNIQUE**

(30) Priority: 01.09.2022 KR 20220110419
(71) Applicant: Dong Woon Anatech Co., Ltd, Seocho-gu Seoul 06713 (KR)
(72) Inventor: JANG, In Su, Seoul 06716 (KR); KWON, Min Su, Seoul 06716 (KR); KWON, Hee Jung, Seoul 06716 (KR); CHUNG, Sung Hwan, Seoul 06716 (KR); IM, Eun Hye, Seoul 06716 (KR); KYE, Ji Won, Seoul 06716 (KR); SHIM, Eun Hyun, Seoul 06716 (KR); KIM, Hee Jin, Seoul 06716 (KR); KIM, Mi Rim, Seoul 06716 (KR); CHO, Hyun Seok, Seoul 06716 (KR); KIM, Dong Cheol, Seoul 06716 (KR)
(74) Representative: V.O.
(86) International application number: PCT/KR2023/012977
(87) International publication number: WO 2024/049234

(57) **Abstract**

It is disclosed a blood glucose prediction system and method using saliva-based artificial intelligence deep learning technique. According to one example embodiment, a postprandial blood glucose prediction system may comprise a learning modeling unit for learning a glucose change inference model to infer a pattern difference between blood glucose change and salivary glucose change according to eating by considering a physical indicator, and learning a postprandial blood glucose inference model to infer a correlation between postprandial salivary glucose and postprandial blood glucose by considering the pattern difference; a target information acquiring unit for acquiring a physical indicator and postprandial salivary glucose of a target; a pattern difference estimating unit for estimating a pattern difference of the target by using the glucose change inference model with the physical indicator of the target as an input parameter; and a postprandial blood glucose predicting unit for predicting postprandial blood glucose of the target by using the postprandial blood glucose inference model with the postprandial salivary glucose and the estimated pattern difference of the target as an input parameter.

## Description

### TECHNICAL FIELD

The below example embodiments relate to a system and method for predicting blood glucose from salivary glucose.

### RELATED ART

As an index for diagnosing diabetes, blood glucose, which is a concentration of glucose in blood, or urine glucose is generally used.

However, in a urine glucose test, since a person with impaired glucose tolerance, which is a borderline type between a diabetic patient and a healthy person, is classified as a diabetic patient, there is a problem in that accuracy of diabetes diagnosis is lowered.

Accordingly, blood glucose, which guarantees the accuracy of diabetes diagnosis, is mainly used. However, to measure such blood glucose, since there is a limitation in which blood can be collected only by an invasive method, disadvantages such as inconvenience, pain, and the like of blood collection may be occurred.

Also, since there is a limitation that the current blood glucose test should be conducted on an empty stomach before meals, there is a disadvantage that an empty stomach should be maintained for the test.

Therefore, there is a need to propose a technology for overcoming the described limitation and constraint of the blood glucose test and solving the disadvantages.

### DETAILED DESCRIPTION

### TECHNICAL SUBJECT

The example embodiments propose a system for predicting postprandial blood glucose from postprandial salivary glucose and diagnosing diabetes from the postprandial blood glucose and a method thereof to overcome a limitation of blood glucose measurement that blood can be collected only by an invasive method and to solve disadvantages such as inconvenience and pain of blood collection, and simultaneously to solve a limitation and disadvantage of maintaining an empty stomach for the test.

In more detail, the example embodiments propose a system for predicting postprandial blood glucose from postprandial salivary glucose and a method thereof by using a glucose change inference model for inferring a pattern difference between blood glucose change and salivary glucose change according to eating by considering a physical indicator and a postprandial blood glucose inference model for inferring a correlation between postprandial salivary glucose and postprandial blood glucose by considering the pattern difference.

At this time, the example embodiments propose a system for improving inference and prediction accuracy and a method thereof, by personalizing and learning the glucose change inference model and the postprandial blood glucose inference model according to a target.

However, technical problems to be solved by the present invention are not limited to the above problems, and may be variously expanded within a scope that does not depart from technical ideas and areas of the present invention.

### SOLUTION

According to one example embodiment, a postprandial blood glucose predication system may include a learning modeling unit for learning a glucose change inference model to infer a pattern difference between blood glucose change and salivary glucose change according to eating by considering a physical indicator, and learning a postprandial blood glucose inference model to infer a correlation between postprandial salivary glucose and postprandial blood glucose by considering the pattern difference; a target information acquiring unit for acquiring a physical indicator and postprandial salivary glucose of a target; a pattern difference estimating unit for estimating a pattern difference of the target by using the glucose change inference model with the physical indicator of the target as an input parameter; and a postprandial blood glucose predicting unit for predicting postprandial blood glucose of the target by using the postprandial blood glucose inference model with the postprandial salivary glucose and the estimated pattern difference of the target as an input parameter.

According to one aspect of one example embodiment, the pattern difference may be configured to include a time delay difference, a maximum value difference, and a rate of change difference between the blood glucose change and the salivary glucose change according to eating.

According to another aspect of one example embodiment, the learning modeling unit may be configured to personalize and learn the glucose change inference model and the postprandial blood glucose inference model according to the target based on personal data including fasting blood glucose of the target.

According to another aspect of one example embodiment, the learning modeling unit may be configured to construct the glucose change inference model by learning experimental data which infers the pattern difference by considering the physical indicator by using an artificial intelligence deep learning technique, and to construct the postprandial blood glucose inference model by learning experimental data which infers the correlation between the postprandial salivary glucose and the postprandial blood glucose by considering the pattern difference by using an artificial intelligence deep learning technique.

According to another aspect of one example embodiment, the physical indicator may be configured to include gender, age, weight, BMI (Body Mass Index), and waist circumference.

According to another aspect of one example embodiment, the learning modeling unit may be configured to learn the glucose change inference model to infer a correlation between the physical indicator and HOMA-IR and HOMA β-cell, and to infer a correlation between the pattern difference and the HOMA-IR and HOMA β-cell.

According to another aspect of one example embodiment, the learning modeling unit may be configured to learn the glucose change inference model to infer a correlation between insulin resistance and insulin secretion calculated by the HOMA-IR and HOMA β-cell and the pattern difference.

According to one example embodiment, a postprandial blood glucose prediction method may include acquiring a physical indicator and postprandial salivary glucose of a target; estimating a pattern difference of the target by using a glucose change inference model -the glucose change inference model is learned to infer a pattern difference between blood glucose change and salivary glucose change according to eating by considering the physical indicator- with the physical indicator of the target as an input parameter; and predicting postprandial blood glucose of the target by using a postprandial blood glucose inference model -the postprandial blood glucose inference model is learned to infer a correlation between postprandial salivary glucose and postprandial blood glucose by considering the pattern difference- with the postprandial salivary glucose and the estimated pattern difference of the target as an input parameter.

According to one aspect of one example embodiment, the pattern difference may be configured to include a time delay difference, a maximum value difference, and a rate of change difference between the blood glucose change and the salivary glucose change according to eating.

According to another aspect of one example embodiment, the glucose change inference model and the postprandial blood glucose inference model may be configured to be personalized and learned according to the target based on personal data including fasting blood glucose of the target.

According to another aspect of one example embodiment, the postprandial blood glucose prediction method may further include constructing the glucose change inference model by learning experimental data which infers the pattern difference by considering the physical indicator by using an artificial intelligence deep learning technique; and constructing the postprandial blood glucose inference model by learning experimental data which infers the correlation between the postprandial salivary glucose and the postprandial blood glucose by considering the pattern difference by using an artificial intelligence deep learning technique.

According to another aspect of one example embodiment, the physical indicator may be configured to include gender, age, weight, BMI (Body Mass Index), and waist circumference.

According to another aspect of one example embodiment, the glucose change inference model may be configured to be learned to infer a correlation between the physical indicator and HOMA-IR and HOMA β-cell, and to infer a correlation between the pattern difference and the HOMA-IR and HOMA β-cell.

According to another aspect of one example embodiment, the glucose change inference model may be configured to be learned to infer a correlation between insulin resistance and insulin secretion calculated by the HOMA-IR and HOMA β-cell and the pattern difference.

According to one example embodiment, a computer-readable medium on which a computer program for executing a postprandial blood glucose prediction method on a computer device is recorded, wherein the postprandial blood glucose prediction method may include acquiring a physical indicator and postprandial salivary glucose of a target; estimating a pattern difference of the target by using a glucose change inference model - the glucose change inference model is learned to infer a pattern difference between blood glucose change and salivary glucose change according to eating by considering the physical indicator- with the physical indicator of the target as an input parameter; and predicting postprandial blood glucose of the target by using a postprandial blood glucose inference model -the postprandial blood glucose inference model is learned to infer a correlation between postprandial salivary glucose and postprandial blood glucose by considering the pattern difference- with the postprandial salivary glucose and the estimated pattern difference of the target as an input parameter.

### EFFECT

The example embodiments may overcome a limitation of blood glucose measurement that blood can be collected only by an invasive method and solve disadvantages such as inconvenience and pain of blood collection, and simultaneously solve a limitation and disadvantage of maintaining an empty stomach for the test by proposing a system for predicting postprandial blood glucose from postprandial salivary glucose and diagnosing diabetes from the postprandial blood glucose and a method thereof.

In more detail, the example embodiments may propose a system for predicting postprandial blood glucose from postprandial salivary glucose and a method thereof by using a glucose change inference model for inferring a pattern difference between blood glucose change and salivary glucose change according to eating by considering a physical indicator and a postprandial blood glucose inference model for inferring a correlation between postprandial salivary glucose and postprandial blood glucose by considering the pattern difference.

At this time, the example embodiments may propose a system for improving inference and prediction accuracy by personalizing and learning the glucose change inference model and the postprandial blood glucose inference model according to a target and a method thereof.

However, effects of the present invention are not limited to the above effects, and may be variously expanded within a scope that does not depart from technical ideas and areas of the present invention.

### BRIEF DESCRIPTION OF THE DRAWINGS

FIG. 1 is a drawing illustrating an example of a diabetes diagnosis environment according to one example embodiment;
FIG. 2 is a block diagram for describing an internal configuration of an electronic device and a server according to one example embodiment;
FIG. 3 is a block diagram illustrating an example of a postprandial blood glucose prediction system according to one example embodiment;
FIG. 4 is a flow chart illustrating a postprandial blood glucose prediction method according to one example embodiment;
FIGS. 5 and 6 are drawings for describing a glucose change inference model used in a postprandial blood glucose prediction method according to one example embodiment.

### BEST MODE

Hereinafter, embodiments of the present invention will be described in detail with reference to the accompanying drawings. However, the present invention is not limited by the embodiments. In addition, same reference numerals on the drawings may refer to same components throughout.

In addition, terminologies used herein are defined to appropriately describe the embodiments of the present invention and thus may be changed depending on a user, the intent of an operator, or a custom in the field to which the present invention pertains. Accordingly, the terminologies must be defined based on the following overall description of this specification. For example, in this specification, singular forms also include plural forms unless specifically stated otherwise in a phrase. In addition, the terms "comprising" and/or "comprises" used in the specification mean that the mentioned elements, steps, operations, and/or devices do not exclude existence or addition of one or more other elements, steps, operations, and/or devices. Also, although terms such as first, second, and the like are used in the specification to describe various areas, directions, shapes, and the like, these areas, directions, and shapes should not be limited by these terms. These terms are only used to distinguish one area, direction, or shape from another area, direction, or shape. Therefore, a part referred to as first part in one example embodiment may be referred to as second part in another example embodiment.

Moreover, it should be understood that various example embodiments of the present invention are not necessarily mutually exclusive although being different from each other. For example, specific shapes, structures, and characteristics described herein may be implemented in other embodiments without departing from technical idea and scope of the present invention in relation to one example embodiment. Besides, it should be understood that the location, arrangement, or configuration of individual components in each of presented categories of an example embodiment may be changed without departing from the technical idea and scope of the present invention.

Hereinafter, in example embodiments, a postprandial blood glucose prediction system and a postprandial blood glucose prediction method for predicting postprandial blood glucose from postprandial salivary glucose and diagnosing diabetes from the postprandial blood glucose.

The postprandial blood glucose prediction method may be performed by at least one computer device that implements a server or an electronic device (e.g., a measuring device or a user terminal connected with the measuring device through communication) which will be described later. Accordingly, the at least one computer device included in the server or electronic device described later may configure the postprandial blood glucose prediction system performing the postprandial blood glucose prediction method. The computer device implementing the postprandial blood glucose predicting system may perform the postprandial blood glucose prediction method according to one example embodiment by control of a driven computer program. The above described computer program may be combined with the computer device and may be stored in a computer-readable medium to execute the postprandial blood glucose prediction method in the computer device. The computer program described herein may have a form of one independent program package or a form that the form of one independent program package is pre-installed in the computer device and linked to an operating system or other program packages.

Also, hereinafter salivary glucose means glucose included in saliva, and blood glucose means glucose included in blood. Also, hereinafter acquiring salivary glucose means acquiring information for salivary glucose (e.g., concentration, index, level, etc. of salivary glucose), and predicting blood glucose means predicting information for blood glucose (e.g., concentration, index, level, etc. of blood glucose).

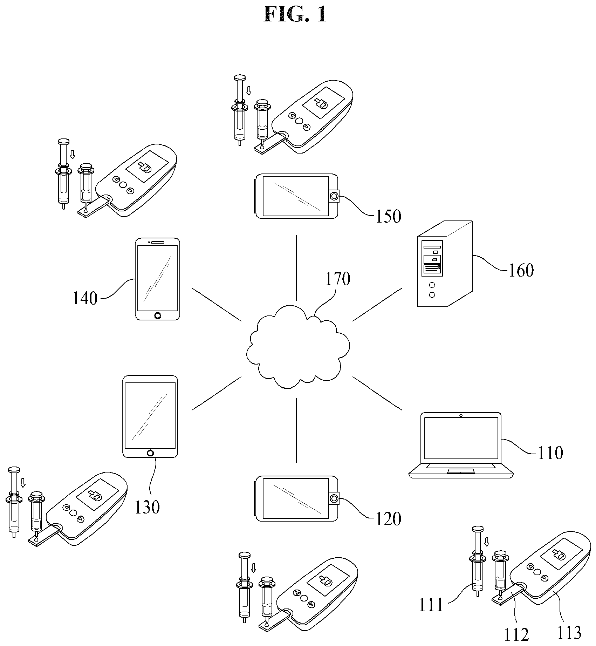

FIG. 1 is a drawing illustrating an example of a diabetes diagnosis environment according to one example embodiment. The diabetes diagnosis environment of FIG. 1 represents an example including a plurality of electronic devices 110, 120, 130, 140, 150, a server 160, and a network 170. Such FIG. 1 is one example for describing the invention, and the number of electronic devices and server is not limited as FIG. 1.

The plurality of electronic devices 110, 120, 130, 140, 150 are terminals possessed by each of specimen targets such as a smart phone, a mobile phone, a tablet PC, a navigation device, a computer, a laptop computer, a terminal for digital broadcasting, a PDA (Personal Digital Assistants), a PMP (Portable Multimedia Player), and the like, and each of the plurality of electronic devices 110, 120, 130, 140, 150 may have a collecting device 111, a biosensor 112, and a measuring device 113.

In other words, each of the specimen targets may possess the collecting device 111, the biosensor 112, and the measuring device 113 with the electronic device 110, and the biosensor 112 and the measuring device 113 may be connected with each of the plurality of electronic devices 110, 120, 130, 140, 150 through a dedicated application installed on each of the plurality of electronic devices 110, 120, 130, 140, 150.

The collecting device 111 may include a specimen collecting unit, a filter, and a compression tube as a device for collecting specimens (e.g., saliva). In a case that the specimen is saliva, the collecting device 111 may remove an interfering substance through the filter in the compression tube from the collected saliva, and provide the saliva from which the interfering substance was removed to the outside, i.e., the biosensor 112.

The biosensor 112, which is an element performing a function of sensing information (e.g., glucose) to be measured from the specimen from which the interfering substance was removed in a case that the specimen (e.g., saliva) collected by the collecting device 111 is inserted through the collecting device 111, may recognize the specimen based on a biosensor strip inserted to the measuring device 113 and a specimen recognizing signal received from the measuring device 113, and provide a responding signal for glucose included in the specimen to the measuring device 113 through a specimen measuring signal applied separately from the specimen recognizing signal.

After the measuring device 113 determines whether the specimen is contacted to the biosensor 112 by providing the specimen recognizing signal to the biosensor 112 by recognizing inserting the biosensor 112, if it is determined that the specimen is contacted, it may measure and indicate a responding signal (responding signal for salivary glucose; e.g., concentration, index, level, etc. of salivary glucose) of the specimen by receiving a responding signal (responding signal for glucose in saliva (salivary glucose)) of the specimen to be measured by providing the specimen measuring signal.

Also, the measuring device 113 may include a communication module in order to communicate with the electronic device (e.g., the first electronic device 110) or the server 160 through the network 170. In such case, the measuring device 113 may predict postprandial blood glucose of the specimen target (hereinafter, referred to as target) based on the responding signal (responding signal for salivary glucose; e.g., concentration, index, level, etc. of salivary glucose) of the specimen in the electronic device 110 or the server 160 by transmitting the responding signal (responding signal for salivary glucose; e.g., concentration, index, level, etc. of salivary glucose) of the specimen to the electronic device 110 or the server 160 and may diagnose diabetes of the target based on the postprandial blood glucose.

However, it is not limited to this, and it may predict postprandial blood glucose of the target based on the responding signal (responding signal for salivary glucose; e.g., concentration, index, level, etc. of salivary glucose) of the specimen in its own measuring device 113 and may diagnose diabetes of the target based on the postprandial blood glucose. In this case, the measuring device 113 may not include the communication module.

The communication method is not limited, and may include short-distance wireless communication between devices in addition to communication methods using a communication network (e.g., a mobile communication network, wired Internet, wireless Internet, and a broadcasting network) that the network 170 may include. For example, the network 170 may include one or more any networks of a PAN (personal area network), a LAN (local area network), a CAN (campus area network), a MAN (metropolitan area network), a WAN (wide area network), a BBN (broadband network), and the Internet. Furthermore, the network 170 may include any one or more of network topologies, including a bus network, a star network, a ring network, a mesh network, a star-bus network, and a tree or hierarchical network, but it is not limited thereto.

The server 160 may receive the responding signal (responding signal for salivary glucose; e.g., concentration, index, level, etc. of salivary glucose) of the specimen measured from the measuring device 113 corresponding to each of the plurality of electronic devices 110, 120, 130, 140, 150 or the plurality of electronic devices 110, 120, 130, 140, 150 by communicating with the plurality of electronic devices 110, 120, 130, 140, 150 through the network 170, and may be implemented with a computer device or a plurality of computer devices for providing a postprandial blood glucose prediction service predicting postprandial blood glucose of the target based on the received responding signal (responding signal for salivary glucose; e.g., concentration, index, level, etc. of salivary glucose) of the specimen and diagnosing diabetes.

In other words, the targets possessing the plurality of electronic devices 110, 120, 130, 140, 150 may be provided with the postprandial blood glucose prediction service that the server 160 provides by connecting to the sever 160 according to control of a pre-installed OS (Operation System) or at least one program (e.g., a browser or the pre-installed application) through the measuring device 113 or the electronic device 110.

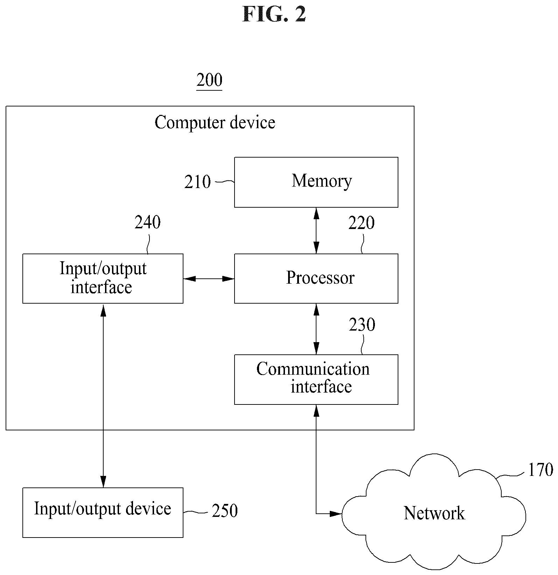

FIG. 2 is a block diagram for describing an internal configuration of an electronic device and a server according to one example embodiment. Each of the plurality of electronic devices 110, 120, 130, 140, 150 or the server 160 described above may be implemented by a computer device 200 illustrated through FIG. 2.

Such computer device 200 may include a memory 210, a processor 220, a communication interface 230, and an input/output interface 240 as shown in FIG. 2. The memory 210 may include a permanent mass storage device such as RAM (random access memory), ROM (read only memory), and disk drive as computer-readable recording medium. Here, the permanent mass storage device such as ROM and disk drive may be included in the computer device 200 as a separate permanent storage device distinct from the memory 210. Also, in the memory 210, an OS and at least one program code may be stored. Such software components may be loaded to the memory 210 from the computer-readable recording medium separate from the memory 210. Such separate computer-readable recording medium may include computer-readable recoding medium such as a floppy drive, a disc, a tape, a DVD/CD-ROM drive, a memory card, and the like. In other example embodiments, the software components may be loaded to the memory 210 through the communication interface 230, not through the computer-readable recording medium. For example, the software components may be loaded to the memory 210 of the computer device 200 based on a computer program installed by files received through the network 170.

The processor 220 may be configured to process computer program instructions by performing basic arithmetic, logic, and input/output operation. The instructions may be provided to the processor 220 by the memory 210 or the communication interface 230. For example, the processor 220 may be configured to execute instructions received according to a program code stored in a recording device such as the memory 210.

The communication interface 230 may provide a function for the computer device 200 to communicate with other devices (e.g. the above described storage devices) with each other through the network 170. For example, a request or an instruction, data, a file, and the like that the processor 220 of the computer device 200 generates according to a program code stored in a recording device such as the memory 210 may be transmitted to other devices through the network 170 according to control of the communication interface 230. Conversely, a signal or an instruction, data, a file, and the like from another device may be received to the computer device 200 through the communication interface 230 of the computer device 200 by going through the network 170. The signal or instruction, data, and the like received through the communication interface 230 may be transmitted to the processor 220 or the memory 210, and the file and the like may be stored in storage medium (the above described permanent storage device) that the computer device 200 may further include.

The input/output interface 240 may be a means for interfacing with an input/output device 250. For example, an input device may include a device such as a microphone, a keyboard or a mouse, and the like, and an output device may include a device such as a display, a speaker, and the like. As another example, the input/output interface 240 may be a means for interfacing with a device in which an input function and an output function are integrated into a single function such as a touch screen.

In addition, in other example embodiments, the computer device 200 may include less or more components than the components in FIG. 2. However, there is no need to clearly illustrate most prior art components. For example, the computer device 200 may be implemented to include at least part of the above described input/output device 250 or may further include other components such as a transceiver, a database, and the like.

Hereinafter, it will be described a specific example embodiment of a postprandial blood glucose prediction method and system for providing a postprandial blood glucose prediction service.

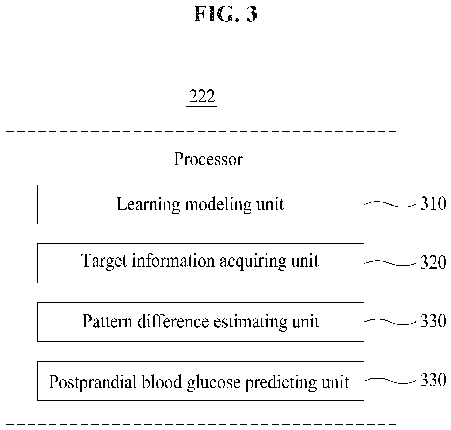

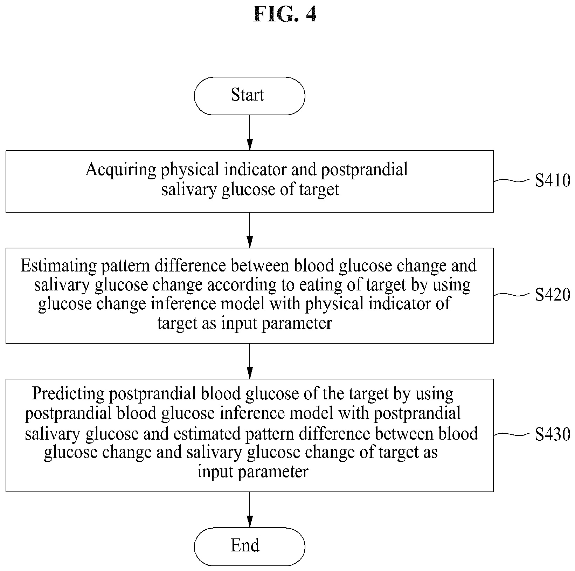

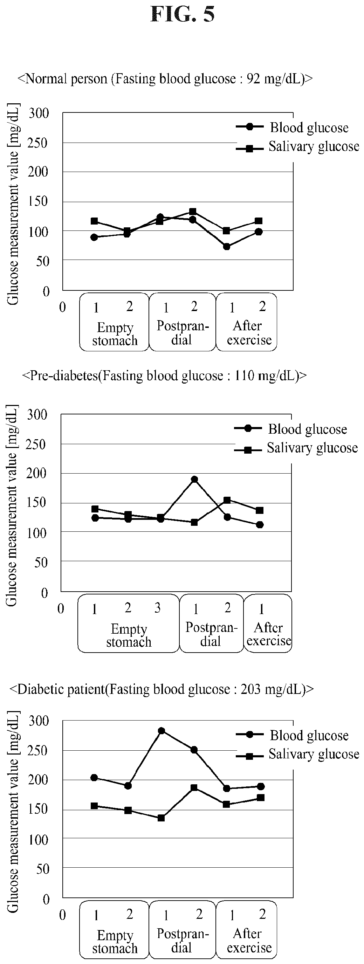

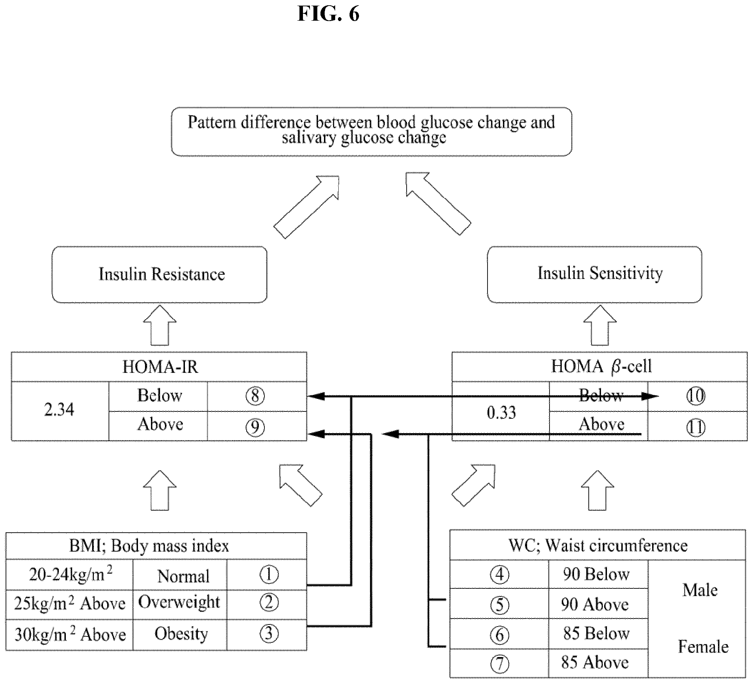

FIG. 3 is a block diagram illustrating an example of a postprandial blood glucose prediction system according to one example embodiment, FIG. 4 is a flow chart illustrating a postprandial blood glucose prediction method according to one example embodiment, and FIGS. 5 and 6 are drawings for describing a glucose change inference model used in a postprandial blood glucose prediction method according to one example embodiment.

In the following example embodiments, the computer device 200 may provide a service for predicting postprandial blood glucose of a target, and based on this, diagnosing diabetes of the target by performing a postprandial blood glucose prediction method described later. For this, in the computer device 200, a postprandial blood glucose prediction system, which is a main subject performing the postprandial blood glucose prediction method, may be configured. For example, the postprandial blood glucose prediction system may be implemented in a form of a program independently operating, or implemented to be configured in a form of in-app of a dedicated application and to be operable on the dedicated application.

The processor 220 of the computer device 200 may be implemented as a component for performing the postprandial blood glucose prediction method according to FIG. 4. For example, the processor 220 may include a learning modeling unit 310, a target information acquiring unit 320, a pattern difference estimating unit 330, and a postprandial blood glucose predicting unit 340 to perform steps S410 to S430 shown in FIG. 4. According to an example embodiment, the components of the processor 220 may be selectively included in or excluded from the processor 220. Also, according to the example embodiment, the components of the processor 220 may be separated or merged for expressions of functions of the processor 220.

Such processor 220 and components of the processor 220 may control the server 160 to perform steps S410 to S430 that the postprandial blood glucose prediction method of FIG. 4 includes. For example, the processor 220 and the components of the processor 220 may be implemented to execute instructions according to a code of an OS and at least one program code that the memory 210 includes.

Here, the components of the processor 220 may be expressions of different functions of the processor 220 performed by the processor 220 according to instructions provided by the program code stored in the server 160. For example, as a functional expression of the processor 220 for predicting postprandial blood glucose of the target, the postprandial blood glucose predicting unit 340 may be used.

The processor 220 may read required instructions from the memory 210 in which instructions related to control of the computer device 200 are loaded. In this case, the read instructions may include instructions for controlling the processor 220 to execute steps S410 to 430 to be described later.

The steps S410 to S420 to be described later may be performed in an order different from the order shown in FIG. 4, and a part of the steps S410 to S430 may be omitted or additional process may be further included.

Before step S410, the learning modeling unit 310 may learn and construct in advance a glucose change inference model with experimental data which infers a pattern difference between blood glucose change and salivary glucose difference according to eating by considering a physical indicator as input data by using an artificial intelligence deep learning technique, and may learn and construct in advance a postprandial blood glucose inference model with experimental data which infers a correlation between postprandial salivary glucose and postprandial blood glucose by considering the pattern difference between the blood glucose change and salivary glucose change according to eating as input data by using an artificial intelligence deep learning technique. The experimental data is clinical test data for numerous specimen targets, and the glucose change inference model and the postprandial blood glucose inference model learned with the experimental data may lead to average inference results and prediction results that satisfy each of various clusters of targets.

A pattern difference between blood glucose change and salivary glucose change is due to a difference of expression of glucose in blood and expression rate and expression degree of glucose in saliva, and it may include a time delay difference, a maximum value difference, and a rate of change difference between the blood glucose change and the salivary glucose change according to eating. For example, blood glucose change and salivary glucose change may have an increased and decreased time delay difference, a maximum glucose level according to increase and decrease, a minimum glucose level difference, and an increased and decreased rate of change difference as shown in FIG. 5 due to difference of expression rate and degree in each of blood and saliva.

Such pattern difference between blood glucose change and salivary glucose change is an index that has great effect on an algorithm for predicting postprandial blood glucose from postprandial salivary glucose, so the postprandial blood glucose inference model used in the postprandial blood glucose prediction method may be learned to infer a correlation between postprandial salivary glucose and postprandial blood glucose by considering the pattern difference between blood glucose change and salivary glucose change according to eating. Accordingly, by using the postprandial blood glucose inference model in the postprandial blood glucose prediction method, the accuracy of predicting postprandial blood glucose from postprandial salivary glucose may be improved.

Meanwhile, the pattern difference between blood glucose change and salivary glucose change according to eating may be affected by a physical indicator. This is because the physical indicator such as gender, age, weight, BMI (Body Mass Index), and waist circumference has a correlation with HOMA-IR and HOMA β-cell, insulin resistance and insulin secretion are calculated by the HOMA-IR and HOMA β-cell, and the pattern difference between blood glucose change and salivary glucose change according to eating has a correlation with the insulin resistance and insulin secretion as shown in FIG. 6.

By using this principle, the learning modeling unit 310 may learn the glucose change inference model to infer a correlation between the physical indicator and the HOMA-IR and HOMA β-cell, and to infer a correlation between the insulin resistance and insulin secretion calculated by the HOMA-IR and HOMA β-cell and the pattern difference between blood glucose change and salivary glucose change according to eating. Therefore, the accuracy that the glucose change inference model infers the pattern difference between blood glucose change and salivary glucose change may be improved.

Particularly, the learning modeling unit 310 may personalize and learn the glucose change inference model and the postprandial blood glucose inference model according to a target based on personal data (e.g., blood glucose change data and salivary glucose change data according to eating based on fasting blood glucose of the target, postprandial salivary glucose data and postprandial blood glucose data of the target, and the like) of the target, so that when the glucose change inference model and the postprandial blood glucose inference model are used, they are specified to the target, so inference results and prediction results having greatly increased accuracy may be caused.

The various publicly known machine-learning algorithms may be used in the learning process of each of the glucose change inference model and the postprandial blood glucose inference model described above.

In step S410, the target information acquiring unit 320 may acquire a physical indicator and postprandial salivary glucose of a target. For example, the target information acquiring unit 320 may receive and acquire information for postprandial saliva (e.g. responding signal for postprandial salivary glucose; e.g., concentration, index, level, etc. of postprandial salivary glucose) measured through the measuring device 113 corresponding to each of the plurality of electronic devices 110, 120, 130, 140, 150 described above, and may receive and acquire a physical indicator of each of targets from the plurality of electronic devices 110, 120, 130, 140, 150.

In step S420, the pattern difference estimating unit 330 may estimate the pattern difference between blood glucose change and salivary glucose change according to eating of a target by using the glucose change inference model with the physical indicator of the target as an input parameter.

In step S430, the postprandial blood glucose predicting unit 340 may predict postprandial blood glucose of the target by using the postprandial blood glucose inference model with the postprandial salivary glucose and the estimated pattern difference between blood glucose change and salivary glucose change according to eating of the target as an input parameter. The estimated postprandial blood glucose of the target may be provided to the electronic device 110 corresponding to the target or the measuring device 113 corresponding to the electronic device 110.

Also, the postprandial blood glucose predicting unit 340 may diagnose diabetes of the target based on the estimated postprandial blood glucose and provide the result. Diagnosing diabetes of the target means confirming whether the target corresponds to a normal person, pre-diabetes, or a diabetic patient.

Like this, the postprandial blood glucose prediction system and method according to one example embodiment may overcome a limitation that blood can be collected only by an invasive method and solve disadvantages such as inconvenience and pain of blood collection, and simultaneously solve a limitation and disadvantage of maintaining an empty stomach for the test by predicting postprandial blood glucose from postprandial salivary glucose by using the glucose change inference model for inferring the pattern difference between blood glucose change and salivary glucose change according to eating by considering the physical indicator and the postprandial blood glucose inference model for inferring the correlation between postprandial salivary glucose and postprandial blood glucose by considering the pattern difference.

Also, the postprandial blood glucose prediction system and method according to one example embodiment may improve inference and prediction accuracy by personalizing and learning the glucose change inference model and the postprandial blood glucose inference model according to a target.

In addition, the predicting postprandial blood glucose from postprandial salivary glucose is described above, but predicting postprandial salivary glucose from postprandial blood glucose may also be possible through the described postprandial blood glucose prediction system and method. In this case, the target information acquiring unit 320 may respond to acquiring blood glucose instead of salivary glucose, and the postprandial blood glucose predicting unit 340 may predict postprandial salivary glucose from the acquired postprandial blood glucose.

The device described herein may be implemented using hardware components, software components, and/or a combination thereof. For example, the device and components described in the example embodiments may be implemented using one or more general-purpose or special purpose computers, such as, for example, a processor, a controller and an ALU (arithmetic logic unit), a digital signal processor, a microcomputer, a FPA (field programmable array), a PLU (programmable logic unit), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will be appreciated that a processing device may include multiple processing elements and/or multiple types of processing elements. For example, a processing device may include multiple processors or a processor and a controller. In addition, different processing configurations are possible, such as parallel processors.

The software may include a computer program, a piece of code, an instruction, or some combination thereof, for independently or collectively instructing or configuring the processing device to operate as desired. Software and/or data may be embodied in any type of machine, component, physical or virtual equipment, computer storage medium or device to provide instructions or data to or being interpreted by the processing device. The software also may be distributed over network coupled computer systems so that the software is stored and executed in a distributed fashion. In particular, the software and data may be stored by one or more computer readable recording mediums.

The method according to the embodiment may be implemented in the form of a program instruction that maybe performed through various computer means, and may be recorded in a computer readable medium. The computer readable medium may include a program instruction, a data file, and a data structure alone or in combination thereof. The program instruction recorded in the medium may be designed or configured particularly for the embodiment or may be a usable one known to those skilled in computer software. An example of the computer readable recording medium may include magnetic media such as a hard disk, a floppy disk, and a magnetic tape, optical recording media such as a CD-ROM and a DVD, magneto-optical media such as a floptical disk, and hardware devices that are particularly configured to store and perform a program instruction, such as a ROM, a RAM, and a flash memory. Further, an example of the program instruction may include high-level language codes which may be executed by a computer using an interpreter as well as machine languages created by using a compiler.

As described above, although the embodiments have been described in connection with the limited embodiments and the drawings, those skilled in the art may modify and change the embodiments in various ways from the description. For example, proper results may be achieved although the aforementioned descriptions are performed in order different from that of the described method and/or the aforementioned elements, such as the system, configuration, device, and circuit, are coupled or combined in a form different from that of the described method or replaced or substituted with other elements or equivalents.

Accordingly, other implementations, other embodiments, and the equivalents of the claims fall within the scope of the claims.

## Claims

1. A postprandial blood glucose prediction system, comprising:
a learning modeling unit for learning a glucose change inference model to infer a pattern difference between blood glucose change and salivary glucose change according to eating by considering a physical indicator, and learning a postprandial blood glucose inference model to infer a correlation between postprandial salivary glucose and postprandial blood glucose by considering the pattern difference;
a target information acquiring unit for acquiring a physical indicator and postprandial salivary glucose of a target;
a pattern difference estimating unit for estimating a pattern difference of the target by using the glucose change inference model with the physical indicator of the target as an input parameter; and
a postprandial blood glucose predicting unit for predicting postprandial blood glucose of the target by using the postprandial blood glucose inference model with the postprandial salivary glucose and the estimated pattern difference of the target as an input parameter.

2. The postprandial blood glucose prediction system of claim 1, wherein the pattern difference is configured to comprise a time delay difference, a maximum value difference, and a rate of change difference between the blood glucose change and the salivary glucose change according to eating.

3. The postprandial blood glucose prediction system of claim 1, wherein the learning modeling unit is configured to personalize and learn the glucose change inference model and the postprandial blood glucose inference model according to the target based on personal data including fasting blood glucose of the target.

4. The postprandial blood glucose prediction system of claim 1, wherein the learning modeling unit is configured to construct the glucose change inference model by learning experimental data which infers the pattern difference by considering the physical indicator by using an artificial intelligence deep learning technique, and to construct the postprandial blood glucose inference model by learning experimental data which infers the correlation between the postprandial salivary glucose and the postprandial blood glucose by considering the pattern difference by using an artificial intelligence deep learning technique.

5. The postprandial blood glucose prediction system of claim 1, wherein the physical indicator is configured to include gender, age, weight, BMI (Body Mass Index), and waist circumference.

6. The postprandial blood glucose prediction system of claim 1, wherein the learning modeling unit is configured to learn the glucose change inference model to infer a correlation between the physical indicator and HOMA-IR and HOMA β-cell, and to infer a correlation between the pattern difference and the HOMA-IR and HOMA β-cell.

7. The postprandial blood glucose prediction system of claim 6, wherein the learning modeling unit is configured to learn the glucose change inference model to infer a correlation between insulin resistance and insulin secretion calculated by the HOMA-IR and HOMA β-cell and the pattern difference.

8. A postprandial blood glucose prediction method, comprising:
acquiring a physical indicator and postprandial salivary glucose of a target;
estimating a pattern difference of the target by using a glucose change inference model -the glucose change inference model is learned to infer a pattern difference between blood glucose change and salivary glucose change according to eating by considering the physical indicator- with the physical indicator of the target as an input parameter; and
predicting postprandial blood glucose of the target by using a postprandial blood glucose inference model -the postprandial blood glucose inference model is learned to infer a correlation between postprandial salivary glucose and postprandial blood glucose by considering the pattern difference- with the postprandial salivary glucose and the estimated pattern difference of the target as an input parameter.

9. The postprandial blood glucose prediction method of claim 8, wherein the pattern difference is configured to comprise a time delay difference, a maximum value difference, and a rate of change difference between the blood glucose change and the salivary glucose change according to eating.

10. The postprandial blood glucose prediction method of claim 8, wherein the glucose change inference model and the postprandial blood glucose inference model is configured to be personalized and learned according to the target based on personal data including fasting blood glucose of the target.

11. The postprandial blood glucose prediction method of claim 8, further comprising:
constructing the glucose change inference model by learning experimental data which infers the pattern difference by considering the physical indicator by using an artificial intelligence deep learning technique, and
constructing the postprandial blood glucose inference model by learning experimental data which infers the correlation between the postprandial salivary glucose and the postprandial blood glucose by considering the pattern difference by using an artificial intelligence deep learning technique.

12. The postprandial blood glucose prediction method of claim 8, wherein the physical indicator is configured to include gender, age, weight, BMI (Body Mass Index), and waist circumference.

13. The postprandial blood glucose prediction method of claim 8, wherein the glucose change inference model is configured to be learned to infer a correlation between the physical indicator and HOMA-IR and HOMA β-cell, and to infer a correlation between the pattern difference and the HOMA-IR and HOMA β-cell.

14. The postprandial blood glucose prediction method of claim 13, wherein the glucose change inference model is configured to be learned to infer a correlation between insulin resistance and insulin secretion calculated by the HOMA-IR and HOMA β-cell and the pattern difference.

15. A computer-readable medium on which a computer program for executing a postprandial blood glucose prediction method on a computer device is recorded, wherein the postprandial blood glucose prediction method comprises:
acquiring a physical indicator and postprandial salivary glucose of a target;
estimating a pattern difference of the target by using a glucose change inference model -the glucose change inference model is learned to infer a pattern difference between blood glucose change and salivary glucose change according to eating by considering the physical indicator- with the physical indicator of the target as an input parameter; and
predicting postprandial blood glucose of the target by using a postprandial blood glucose inference model -the postprandial blood glucose inference model is learned to infer a correlation between postprandial salivary glucose and postprandial blood glucose by considering the pattern difference- with the postprandial salivary glucose and the estimated pattern difference of the target as an input parameter.
